# EUROPEAN PATENT APPLICATION

(11) **EP 4 745 141 A1**
(43) Date of publication of application: **20.05.2026**
(21) Application number: 24818827.8
(22) Date of filing: 18.07.2024
(51) Int. Cl.: C07D 487/04

(54) **METHOD FOR PREPARING INTERMEDIATE OF ADRENERGIC RECEPTOR AGONIST**

(30) Priority: 08.06.2023 CN 202310673643
(71) Applicant: Porton Pharma Solutions Ltd., Chongqing 401220 (CN)
(72) Inventor: LIN, Wenqing, Chongqing 401220 (CN); LI, Jianguo, Chongqing 401220 (CN); XU, Huan, Chongqing 401220 (CN); LIU, Shikui, Chongqing 401220 (CN); ZHENG, Hongjie, Chongqing 401220 (CN); LEI, Xiaoli, Chongqing 401220 (CN); ZHANG, Jing, Chongqing 401220 (CN)
(74) Representative: Scholl, Matthias
(86) International application number: PCT/CN2024/106105
(87) International publication number: WO 2024/251302

(57) **Abstract**

A method for preparing an intermediate of an adrenergic receptor agonist. The method comprises the following steps: cyclizing the compound of formula E1 or the compound of formula E2 and the compound of formula F0 to obtain the compound of formula C, wherein R₁ is methyl, ethyl, isopropyl, propyl, tert-butyl, or butyl; R₃ is H, Cl, F or Br; and L₁ and L₂ are each individually a leaving group. The method uses easily available raw materials, avoids the use of toxic and harmful reagents, is mild in terms of reaction conditions, is simple to operate, achieves a substantial improvement in yield and reduction in cost, and is more favorable for commercial production.

## Description

The disclosure relates to the field of pharmaceutical manufacturing, and more particularly, to a method for preparing an intermediate of an adrenergic receptor agonist.

Vibegron is a selective human beta-3 adrenergic receptor agonist. Activation of the beta-3 adrenergic receptor relaxes detrusor smooth muscle during bladder filling, increasing bladder capacity and reducing symptoms of overactive bladder (OAB) in adults.

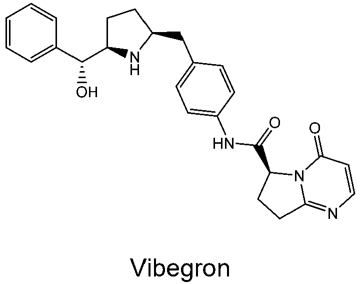

Compounds (S)-4-oxo-4,6,7,8-tetrahydropyrrolo(1,2-a)pyrimidine-6-carboxylic acid (Compound D1) and its sodium salt (Compound D2) are critical intermediates for the synthesis of Vibegron. These are typically obtained by hydrolyzing Compound C under acidic or basic conditions.

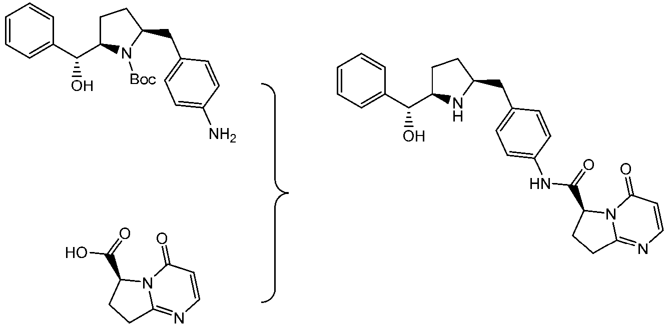

PCT Patent Publication No. WO2013062881A1 discloses a synthetic route for the intermediates. This route involves reacting a starting material RM1 with dimethyl sulfate to form Compound A, and reacting RM2 with chlorosulfonyl isocyanate to form Compound B. The compounds A and B are then reacted in ethylbenzene solvent at a temperature of 128°C for 48 hours to yield a target compound C.

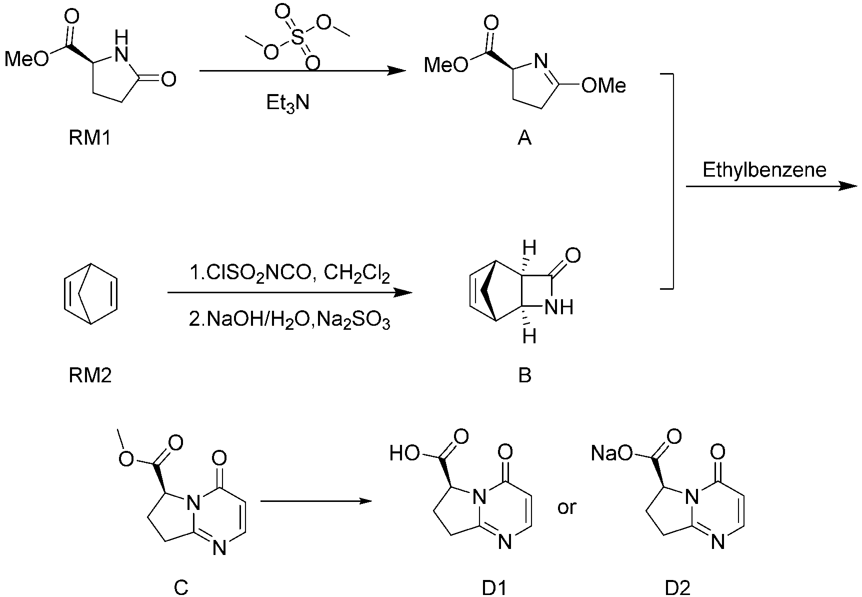

The synthesis process suffers from the following disadvantages:
1. RM2 is an expensive raw material, leading to high product costs.
2. Chlorosulfonyl isocyanate is highly corrosive, posing substantial safety risks during handling and production.
3. The reaction between A and B to form C generates cyclopentadiene as a byproduct, which is environmentally unfriendly and hazardous to operators. The high reaction temperature also promotes polymerization, resulting in large amounts of tar-like polymers that adhere to equipment, complicating scale-up, separation, and purification.
4. The prepared product C requires column chromatography for purification, which is unsuitable for commercial-scale production and results in low overall yield.

The disclosure provides a method for preparing an intermediate of an adrenergic receptor agonist that overcomes limitations in the prior art. The disclosed method utilizes readily available starting materials, avoids toxic or hazardous reagents, and employs mild reaction conditions. This process is simple to operate, provides a substantially improved yield, reduces overall cost, and is well-suited for commercial-scale production.

The disclosure provides a method for preparing an intermediate of an adrenergic receptor agonist, comprising contacting a first compound of formula E1 or E2 with a second compound of formula F0 to yield a third compound of formula C: 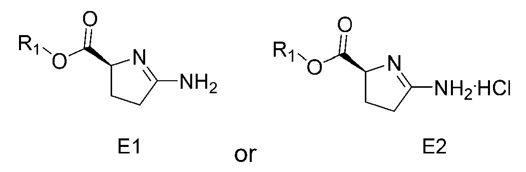 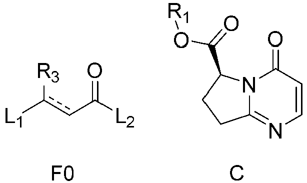
where, R₁ is selected from the group consisting of methyl, ethyl, isopropyl, propyl, tert-butyl, and butyl;
R₃ is selected from the group consisting of H, Cl, F, and Br; and
L₁ and L₂ are each independently a leaving group.

In a class of this embodiment, the formula F0 comprises a compound of formula F: 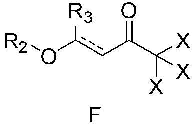
R₂ is selected from the group consisting of methyl, ethyl, isopropyl, propyl, tert-butyl, and butyl;
R₃ is selected from the group consisting of H, Cl, F, and Br; and
X is selected from the group consisting of F, Cl, and Br.

In a class of this embodiment, the compound of formula F0 comprises a compound of formula F-1: 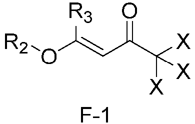
R₂ is selected from the group consisting of methyl, ethyl, isopropyl, propyl, tert-butyl, and butyl;
R₃ is H; and
X is selected from the group consisting of F, Cl, and Br.

In a class of this embodiment, the compound of formula F-1 comprises the following compounds:

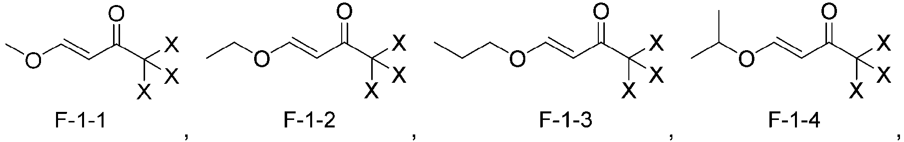

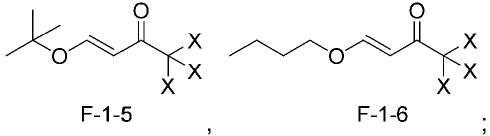

X is selected from the group consisting of F, Cl, and Br.

In a class of this embodiment, the compound of formula F0 comprises a compound of formula F-2: 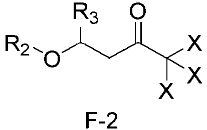
R₂ is selected from the group consisting of methyl, ethyl, isopropyl, propyl, tert-butyl, and butyl;
R₃ is selected from the group consisting of F, Cl, and Br; and
X is selected from the group consisting of F, Cl, and Br.

In a class of this embodiment, the compound of formula F-2 comprises the following compounds: 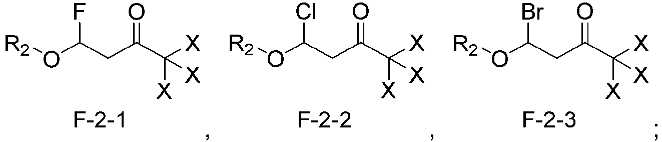
R₂ is selected from the group consisting of methyl, ethyl, isopropyl, propyl, tert-butyl, and butyl; and
X is selected from the group consisting of F, Cl, and Br.

In a class of this embodiment, the compound of formula F-2-1 comprises the following compounds:

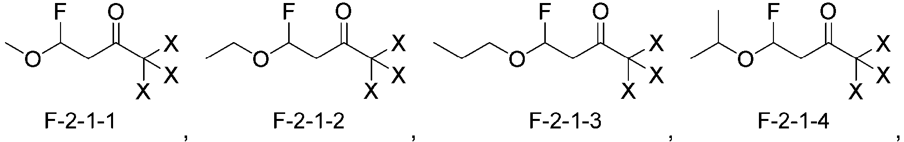

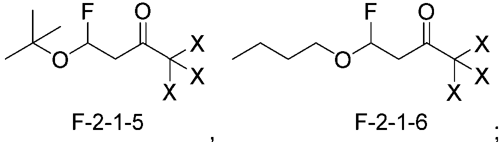

X is selected from the group consisting of F, Cl, and Br.

In a class of this embodiment, the compound of formula F-2-2 comprises the following compounds:

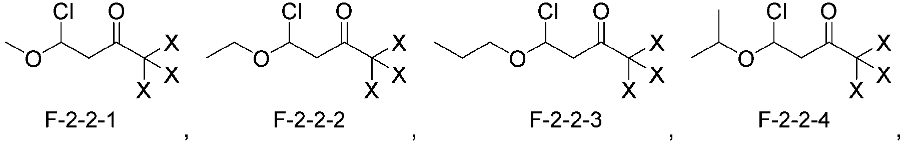

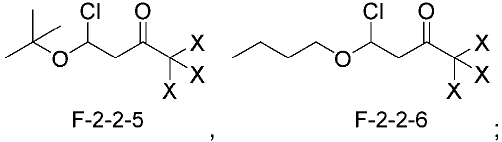

X is selected from the group consisting of F, Cl, and Br.

In a class of this embodiment, the compound of formula F-2-3 comprises the following compounds:

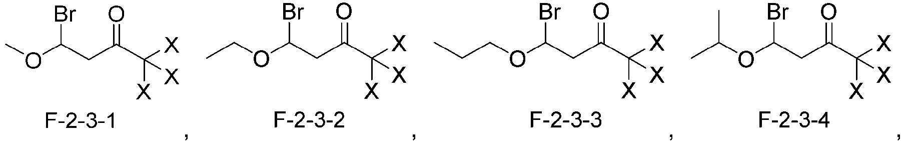

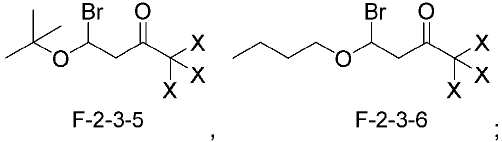

X is selected from the group consisting of F, Cl, and Br.

In a class of this embodiment, the formula F comprises a compound of formula F₁:

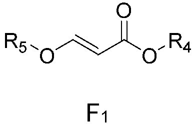

R₄ and R₅ are each independently C₁-C₆ alkyl.

In a class of this embodiment, R₄ and R₅ of the compound of formula F₁ are each independently selected from the group consisting of methyl, ethyl, propyl, butyl, isopropyl, and tert-butyl.

In a class of this embodiment, the formula F comprises a compound of formula F₂:

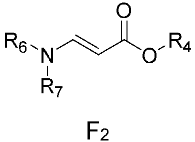

R₄, R₆, and R₇ are each independently C₁-C₆ alkyl.

In a class of this embodiment, R₄, R₆, and R₇ of the compound of formula F₂ are each independently selected from the group consisting of methyl, ethyl, propyl, butyl, isopropyl, and tert-butyl.

In a class of this embodiment, the first compound of formula E1 or E2 reacts with the second compound of formula F0 in the presence of a base.

In a class of this embodiment, the base is an organic base or an inorganic base.

In a class of this embodiment, the organic base is selected from the group consisting of triethylamine, pyridine, N,N-diisopropylethylamine, and 4-dimethylaminopyridine.

In a class of this embodiment, the organic base is triethylamine or N,N-diisopropylethylamine.

In a class of this embodiment, the compound of formula E1 or E2, the compound of formula F0, and the base are mixed in a molar ratio in the range of 1.0: from 0.5 to 3.0: from 0.5 to 5.0, and more specifically 1.0: from 1.0 to 1.5: from 1.0 to 3.0.

In a class of this embodiment, the molar ratio of the compound of formula E1, the compound of formula F-1, and the base is 1.0: from 0.5 to 2.0: from 0.5 to 2.0, and more specifically 1.0: from 1.0 to 1.5: from 1.0 to 1.5.

In a class of this embodiment, the molar ratio of the compound of formula E1, the compound of formula F-2, and the base is 1.0: from 0.5 to 2.0: from 1.5 to 4.0, and more specifically 1.0: from 1.0 to 1.5: from 1.5 to 3.0.

In a class of this embodiment, the molar ratio of the compound of formula E1, the compound of formula F₁, and the base is 1.0: from 0.5 to 2.0: from 0.5 to 2.0, and more specifically 1.0: from 1.0 to 1.5: from 1.0 to 1.5;
the molar ratio of the compound of formula E2, the compound of formula F₁, and the base is 1.0: from 0.5 to 2.0: from 0.5 to 3.0, and more specifically 1.0: from 1.0 to 1.5: from 1.5 to 2.0;
the molar ratio of the compound of formula E1, the compound of formula F₂, and the base is 1.0: from 0.5 to 2.0: from 0.5 to 2.0, and more specifically 1.0: from 1.0 to 1.5: from 1.0 to 1.5;
the molar ratio of the compound of formula E2, the compound of formula F₂, and the base is 1.0: from 0.5 to 2.0: from 0.5 to 3.0, and more specifically 1.0: from 1.0 to 1.5: from 1.5 to 2.0.

In a class of this embodiment, the reaction temperature ranges from 25°C to 120°C, and more specifically from 50°C to 110°C.

In a class of this embodiment, an organic solvent is selected from the group consisting of ethyl acetate, isopropyl acetate, toluene, and acetonitrile.

The following advantages are associated with the method for preparing an intermediate of an adrenergic receptor agonist of the disclosure.

The synthetic route employed in the method is conducted under mild reaction conditions and features simple operation, making the method suitable for scale-up and commercial production. The materials used are highly safe, and the process does not generate by-products that are harmful to the environment or human health, aligning with the requirements of green and sustainable development.

The starting materials utilized in the route of the method are inexpensive and readily available, resulting in low product costs and providing a significant competitive price advantage.

The process of the disclosure provides a higher product yield compared to the existing prior art.
FIG. 1 is an HPLC chromatogram of an intermediate prepared in Example 1;
FIG. 2 is an HPLC chromatogram depicting the chiral purity of an intermediate prepared in Example 1; and
FIG. 3 is a 1H NMR spectrum of an intermediate prepared in Example 1.

To further illustrate the disclosure, embodiments detailing a method for preparing an intermediate of an adrenergic receptor agonist are described below. It should be noted that the following embodiments are intended to describe and not to limit the disclosure.

In one embodiment, a first compound of formula E1 or E2 reacts with a second compound of formula F0, as shown in Reaction formula I:

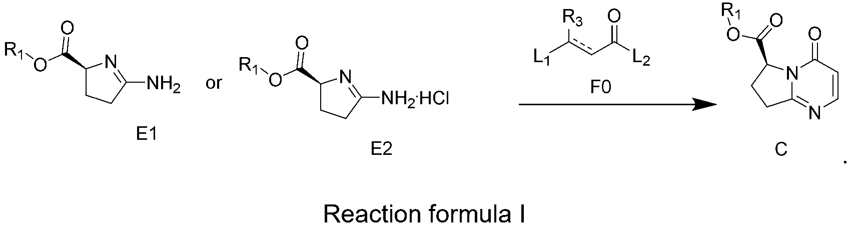

The reaction is carried out in the presence of a base. Suitable bases include organic bases and inorganic bases. Non-limiting examples of inorganic bases include sodium carbonate, potassium carbonate, sodium bicarbonate, potassium bicarbonate, sodium hydroxide, and potassium hydroxide. Non-limiting examples of organic bases include triethylamine, pyridine, N,N-diisopropylethylamine, and 4-dimethylaminopyridine. In some embodiments, the base is triethylamine or N,N-diisopropylethylamine.

The reactants and the base are employed in a molar ratio of the first compound of formula E1 or E2 to the second compound of formula F0 to the base in the range of 1.0: 0.5-3.0: 0.5-5.0. A preferable molar ratio is 1.0: 1.0-1.5: 1.0-4.0.

The reaction is conducted in an organic solvent. Suitable solvents include, but are not limited to, ethyl acetate, isopropyl acetate, toluene, and acetonitrile. Ethyl acetate and toluene are preferred.

The reaction temperature is typically maintained within the range of from 25°C to 120°C. A further suitable temperature range is from 50°C to 110°C. Specific exemplary reaction temperatures include, for example, 50°C, 55°C, 60°C, 65°C, 70°C, 75°C, 80°C, 85°C, 90°C, 95°C, 100°C, 105°C, 110°C, 115°C, and 120°C.

The first compound of formula E1 includes but is not limited to the following compounds:

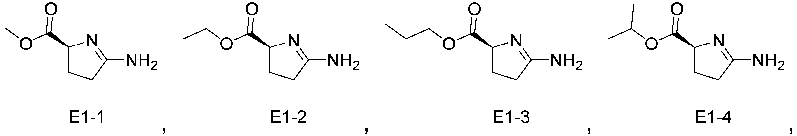

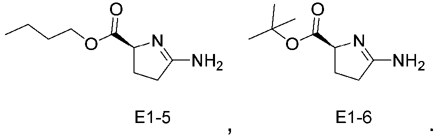

The first compound of formula E2 includes but is not limited to the following compounds:

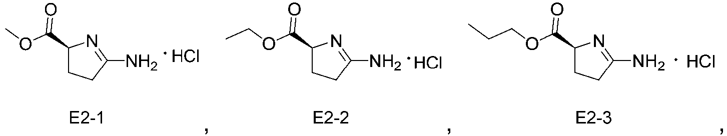

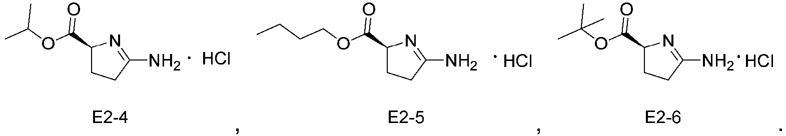

The synthesis of the compound of formula C is achieved through a cyclization reaction. This process involves reacting the compound of formula F0 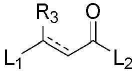 with the compound of either formula E1 or formula E2. As a result of this reaction, a compound of formula C is formed. In this cyclization, the compound of formula F0, which features a saturated or unsaturated carbon chain, serves as a key building block. Specifically, three carbon atoms from this carbon chain incorporate into the growing heterocyclic system during the reaction with the formula E1 or E2, thereby constructing the dihydropyrrolopyrimidine ring core of the final derivative.

In certain embodiments, the compound of formula F0 is a substituted butan-2-one:

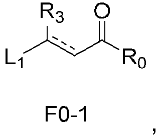

R₀ represents three halogen substituents, each independently selected from the group consisting of F, Cl, and Br.

In certain embodiments, the compound of formula F0 is 1,1,1-trihalobutan-2-one:

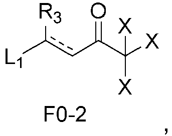

In certain embodiments, the compound of formula F0 is 1,1,1-trihalo-4-(oxoalkyl)butan-2-one: 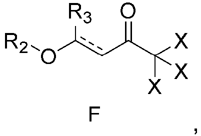 the leaving groups L₁ and L₂ are an oxoalkyl group 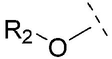 and a 1,1,1-trihalomethyl group, respectively.

In certain embodiments, the compound of formula F comprises a compound of formula F-1: 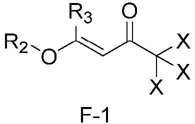
R₂ is selected from the group consisting of methyl, ethyl, isopropyl, propyl, tert-butyl, and butyl;
R₃ is H; and
X is selected from the group consisting of F, Cl, and Br.

Furthermore, the compound of formula F-1 comprises the following compounds:

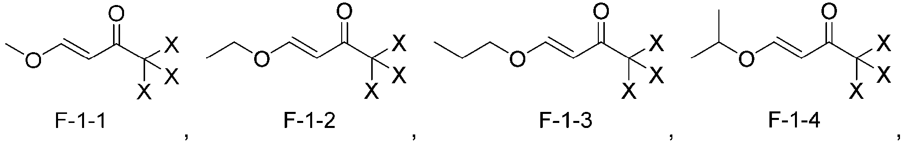

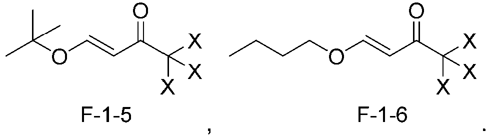

In certain embodiments, the compound of formula F comprises a compound of formula F-2: 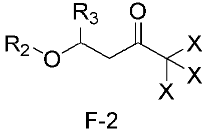
R₂ is selected from the group consisting of methyl, ethyl, isopropyl, propyl, tert-butyl, and butyl;
R₃ is selected from the group consisting of F, Cl, and Br; and
X is selected from the group consisting of F, Cl, and Br.

Furthermore, the compound of formula F-2 comprises the following compounds: 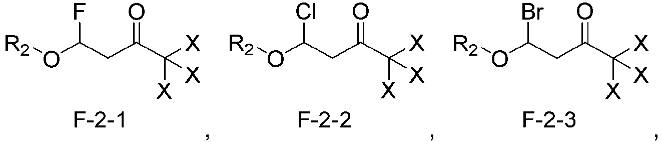
R₂ is selected from the group consisting of methyl, ethyl, isopropyl, propyl, tert-butyl, and butyl; and
X is selected from the group consisting of F, Cl, and Br.

Furthermore, the compound of formula F-2-1 comprises the following compounds:

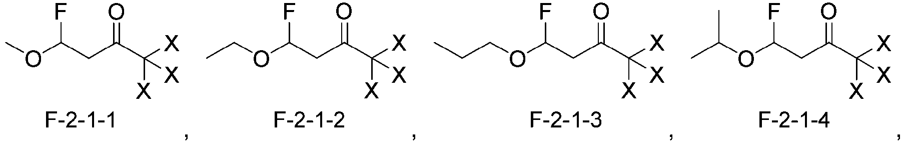

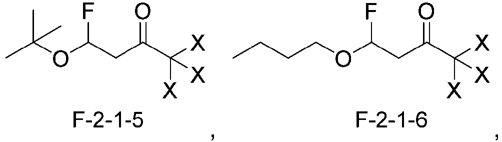

X is selected from the group consisting of F, Cl, and Br.

Furthermore, the compound of formula F-2-2 comprises the following compounds:

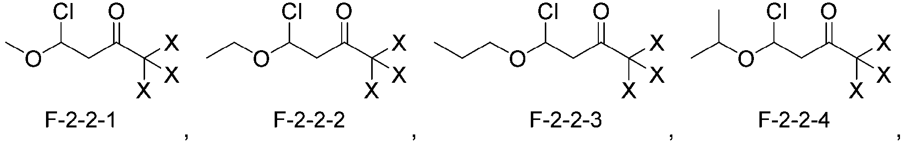

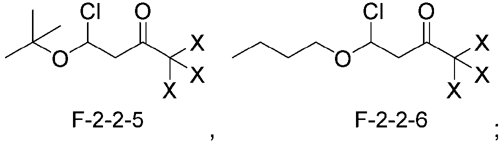

X is selected from the group consisting of F, Cl, and Br.

Furthermore, the compound of formula F-2-3 comprises the following compounds:

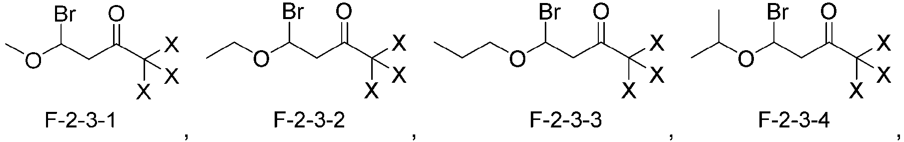

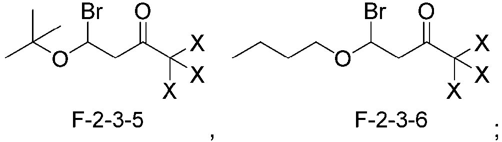

In certain embodiments, when R₃ of the compound of formula F0 is H, F0 is a substituted acrylate.

L₁ and L₂ are leaving groups; L₁ is an oxoalkyl group 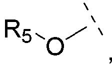 and L₂ is

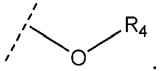

Furthermore, the formula F comprises a compound of formula F₁:

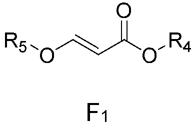

R₄ and R₅ are each independently C₁-C₆ alkyl.

Furthermore, R₄ and R₅ of the compound of formula F₁ are each independently selected from the group consisting of methyl, ethyl, propyl, butyl, isopropyl, and tert-butyl.

Furthermore, the compound of formula F1 is as follows:

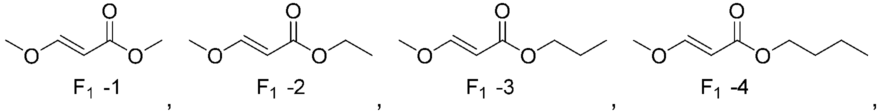

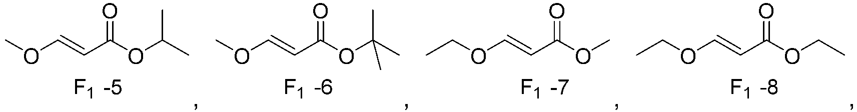

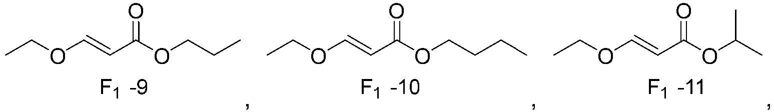

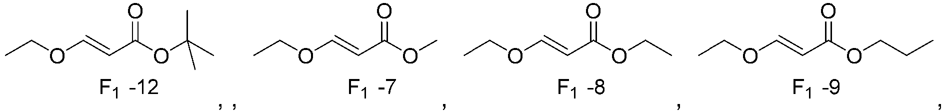

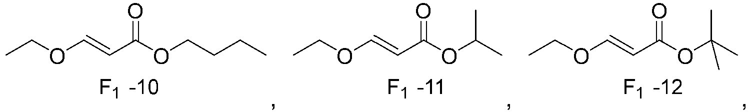

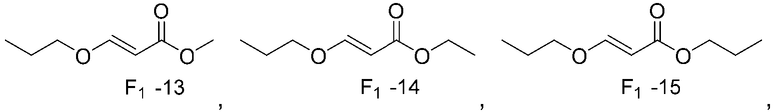

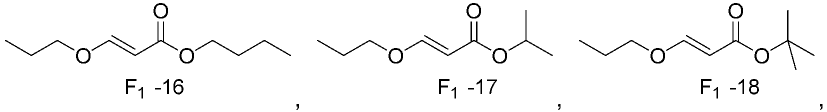

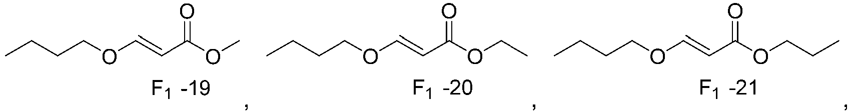

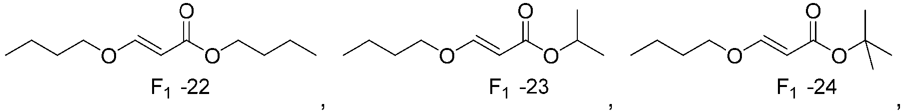

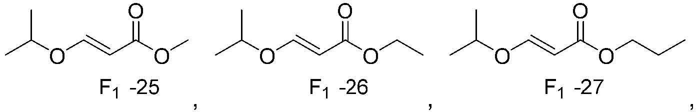

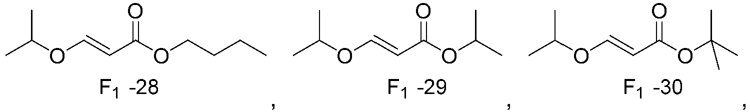

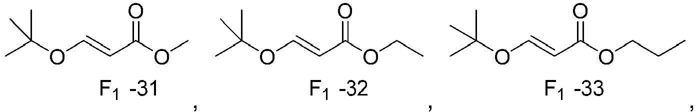

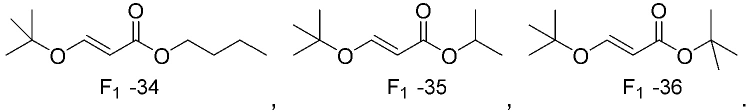

Furthermore, when R₃ of the compound of formula F0 is H, F0 is a substituted acrylate.

L₁ and L₂ are leaving groups; L₁ is an oxoalkyl group 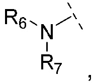 and L₂ is

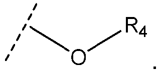

Furthermore, the formula F comprises a compound of formula F₂:

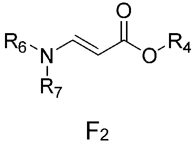

R₄, R₆, and R₇ are each independently C₁-C₆ alkyl.

Furthermore, R₄, R₆, and R₇ of the compound of formula F₂ are each independently selected from the group consisting of methyl, ethyl, propyl, butyl, isopropyl, and tert-butyl.

Furthermore, when R₄ of the compound of formula F₂ is selected from the group consisting of methyl, ethyl, propyl, butyl, isopropyl, and tert-butyl, R₆ and R₇ of the compound of formula F₂ are each independently selected from the group consisting of methyl, ethyl, propyl, butyl, isopropyl, and tert-butyl.

Preferably, the compound of formula F₂ is as follows:

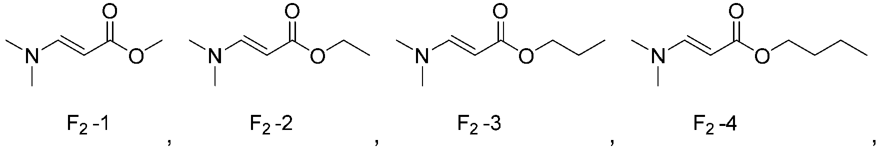

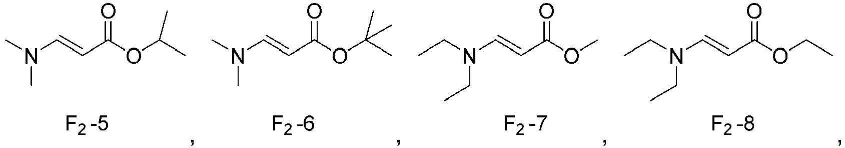

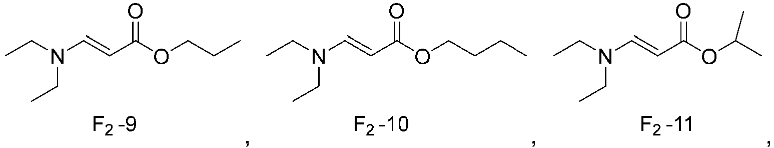

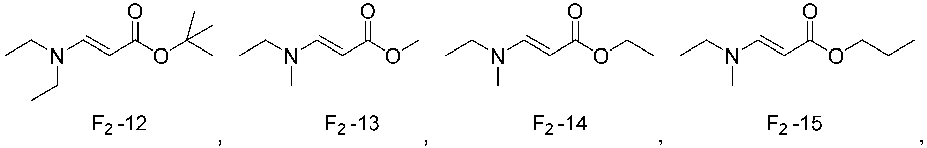

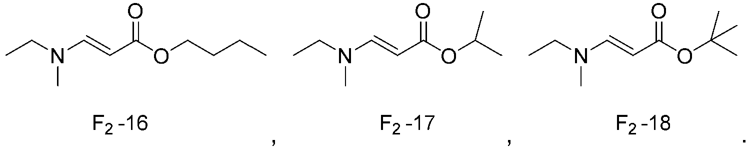

Furthermore, the first compound of formula E1 or E2 reacts with a compound of formula F-1 is shown in Reaction formula 2:

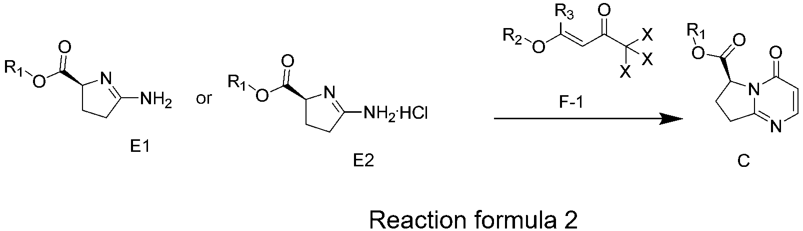

In certain embodiments, R₃ is H.

The reaction formula 2 is carried out in the presence of a base. The base is an organic base or an inorganic base. Non-limiting examples of suitable inorganic bases include sodium carbonate, potassium carbonate, sodium bicarbonate, potassium bicarbonate, sodium hydroxide, and potassium hydroxide. Suitable organic bases include, but are not limited to, triethylamine, pyridine, N,N-diisopropylethylamine, and 4-dimethylaminopyridine. In some embodiments, the base is triethylamine or N,N-diisopropylethylamine.

Furthermore, the compound of formula E1 or E2, the compound of formula F-1, and the base are mixed in a molar ratio in the range of 1.0: from 0.5 to 2.0: from 0.5 to 2.0, and more specifically 1.0: from 1.0 to 1.5: from 1.0 to 2.0.

For the compound of formula E1, the compound of formula F-1, and the base, the molar ratio is 1.0: from 0.5 to 2.0: from 0.5 to 2.0, more specifically 1.0: from 1.0 to 1.5: from 1.0 to 1.5, and even more specifically selected from the group consisting of 1.0: 1.0: 1.0, 1.0: 1.5: 1.0, 1.0: 1.0: 2.0, 1.0: 1.5: 2.0, and 1.0: 1.2: 1.5.

For the compound of formula E2, the compound of formula F-1, and the base, the molar ratio is 1.0: from 0.5 to 2.0: from 0.5 to 3.0, more specifically 1.0: from 1.0 to 1.5: from 1.5 to 2.0, and even more specifically selected from the group consisting of 1.0: 1.0: 1.0, 1.0: 1.5: 1.0, 1.0: 1.0: 2.0, 1.0: 1.5: 2.0, and 1.0: 1.2: 1.5.

The reaction solvent comprises ethyl acetate, isopropyl acetate, toluene, and acetonitrile, preferably ethyl acetate or toluene.

The reaction temperature is from 25°C to 120°C, more specifically from 50°C to 110°C. In further embodiments, the reaction temperature is selected from the group consisting of 50°C, 55°C, 60°C, 65°C, 70°C, 75°C, 80°C, 85°C, 90°C, 95°C, 100°C, 105°C, 110°C, 115°C, and 120°C.

The first compound of formula E1 or E2 reacts with a compound of formula F-2 is shown in Reaction formula 3:

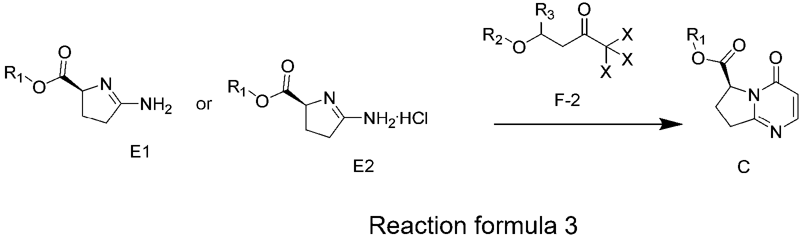

In certain embodiments, R₃ is F, Cl, or Br.

The reaction formula 3 is carried out in the presence of a base. The base is an organic base or an inorganic base. Non-limiting examples of suitable inorganic bases include sodium carbonate, potassium carbonate, sodium bicarbonate, potassium bicarbonate, sodium hydroxide, and potassium hydroxide. Suitable organic bases include, but are not limited to, triethylamine, pyridine, N,N-diisopropylethylamine, and 4-dimethylaminopyridine. In some embodiments, the base is triethylamine or N,N-diisopropylethylamine.

The compound of formula E1 or E2, the compound of formula F-2, and the base are mixed in a molar ratio in the range of 1.0: from 0.5 to 2.0: from 1.5 to 5.0, and more specifically 1.0: from 1.0 to 1.5: from 1.5 to 4.0.

For the compound of formula E1, the compound of formula F-2, and the base, the molar ratio is 1.0: from 0.5 to 2.0: from 1.5 to 4.0, more specifically 1.0: from 1.0 to 1.5: from 2.0 to 3.0, and even more specifically selected from the group consisting of 1.0: 1.0: 2.0, 1.0: 1.0: 3.0, 1.0: 1.5: 2.0, 1.0: 1.0: 2.5, and 1.0: 1.5: 2.0.

For the compound of formula E2, the compound of formula F-2, and the base, the molar ratio is 1.0: from 0.5 to 2.0: from 0.5 to 5.0, more specifically 1.0: from 1.0 to 1.5: from 2.5 to 4.0, and even more specifically selected from the group consisting of 1.0: 1.0: 3.0, 1.0: 1.5: 3.0, 1.0: 1.0: 2.5, 1.0: 1.0: 4.0, 1.0: 1.5: 2.5, and 1.0: 1.5: 4.0.

The reaction solvent comprises ethyl acetate, isopropyl acetate, toluene, and acetonitrile, preferably ethyl acetate or toluene.

The reaction temperature is from 25°C to 120°C, more specifically from 50°C to 110°C. In further embodiments, the reaction temperature is selected from the group consisting of 50°C, 55°C, 60°C, 65°C, 70°C, 75°C, 80°C, 85°C, 90°C, 95°C, 100°C, 105°C, 110°C, 115°C, and 120°C.

Furthermore, the first compound of formula E1 or E2 reacts with a compound of formula F₁ is shown in Reaction formula 4:

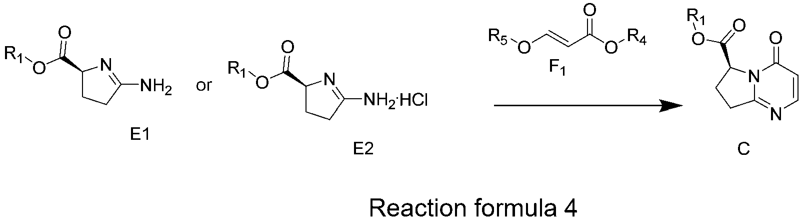

The reaction formula 4 is carried out in the presence of a base. The base is an organic base or an inorganic base. Non-limiting examples of suitable inorganic bases include sodium carbonate, potassium carbonate, sodium bicarbonate, potassium bicarbonate, sodium hydroxide, and potassium hydroxide. Suitable organic bases include, but are not limited to, triethylamine, pyridine, N,N-diisopropylethylamine, and 4-dimethylaminopyridine. In some embodiments, the base is triethylamine or N,N-diisopropylethylamine.

The compound of formula E1 or E2, the compound of formula F₁, and the base are mixed in a molar ratio in the range of 1.0: from 0.5 to 2.0: from 0.5 to 2.0, and more specifically 1.0: from 1.0 to 1.5: from 1.0 to 2.0.

For the compound of formula E1, the compound of formula F₁, and the base, the molar ratio is 1.0: from 0.5 to 2.0: from 0.5 to 2.0, and more specifically 1.0: from 1.0 to 1.5: from 1.0 to 1.5.

For the compound of formula E2, the compound of formula F₁, and the base, the molar ratio is 1.0: from 0.5 to 2.0: from 0.5 to 3.0, and more specifically 1.0: from 1.0 to 1.5: from 1.5 to 2.0.

The reaction solvent comprises ethyl acetate, isopropyl acetate, toluene, and acetonitrile, preferably ethyl acetate or toluene.

The reaction temperature is from 25°C to 120°C, more specifically from 50°C to 110°C. In further embodiments, the reaction temperature is selected from the group consisting of 50°C, 55°C, 60°C, 65°C, 70°C, 75°C, 80°C, 85°C, 90°C, 95°C, 100°C, 105°C, 110°C, 115°C, and 120°C.

Furthermore, the first compound of formula E1 or E2 reacts with a compound of formula F₂ is shown in Reaction formula 5:

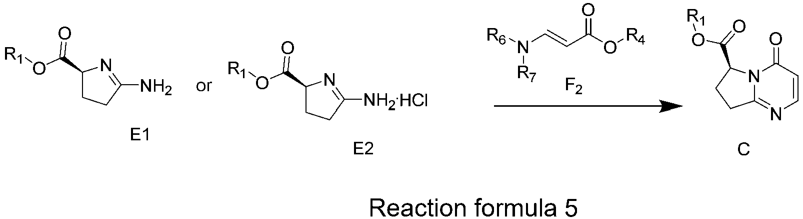

The reaction formula 5 is carried out in the presence of a base. The base is an organic base or an inorganic base. Non-limiting examples of suitable inorganic bases include sodium carbonate, potassium carbonate, sodium bicarbonate, potassium bicarbonate, sodium hydroxide, and potassium hydroxide. Suitable organic bases include, but are not limited to, triethylamine, pyridine, N,N-diisopropylethylamine, and 4-dimethylaminopyridine. In some embodiments, the base is triethylamine or N,N-diisopropylethylamine.

The compound of formula E1 or E2, the compound of formula F₂, and the base are mixed in a molar ratio in the range of 1.0: from 0.5 to 2.0: from 0.5 to 2.0, and more specifically 1.0: from 1.0 to 1.5: from 1.0 to 2.0.

For the compound of formula E1, the compound of formula F₂, and the base, the molar ratio is 1.0: from 0.5 to 2.0: from 0.5 to 2.0, and more specifically 1.0: from 1.0 to 1.5: from 1.0 to 1.5.

For the compound of formula E2, the compound of formula F₂, and the base, the molar ratio is 1.0: from 0.5 to 2.0: from 0.5 to 3.0, and more specifically 1.0: from 1.0 to 1.5: from 1.5 to 2.0.

The reaction solvent comprises ethyl acetate, isopropyl acetate, toluene, and acetonitrile, preferably ethyl acetate or toluene.

The reaction temperature is from 25°C to 120°C, more specifically from 50°C to 110°C. In further embodiments, the reaction temperature is selected from the group consisting of 50°C, 55°C, 60°C, 65°C, 70°C, 75°C, 80°C, 85°C, 90°C, 95°C, 100°C, 105°C, 110°C, 115°C, and 120°C.

### Example 1

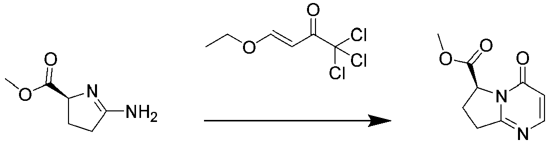

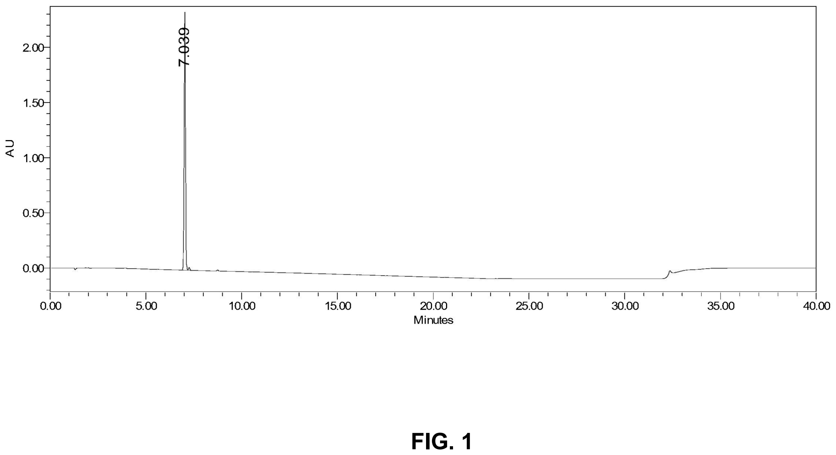

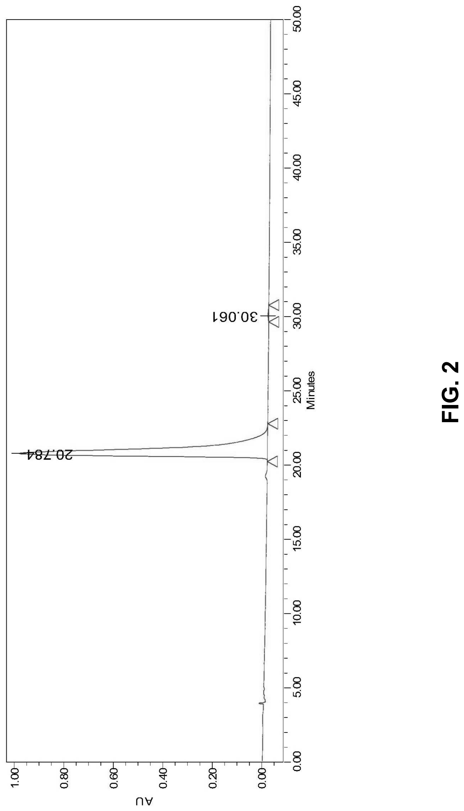

Compound E1 ((S)-5-amino-3,4-dihydro-2H-pyrrole-2-carboxylic acid methyl ester) (14.2 g, 99.9 mmol), isopropyl acetate, Compound F-1 (1,1,1-trichloro-4-ethoxy-3-ene-2-one) (21.8 g, 100.2 mmol), and triethylamine (10.2 g, 100.8 mmol) were successively charged into a reaction vessel. The mixture was heated to about 90°C to react. After the reaction was completed, the mixture was cooled to room temperature. Water was added to quench the reaction, and the phases were separated. The aqueous phase was extracted with isopropyl acetate. The combined organic phases were dried over anhydrous sodium sulfate, filtered, and the filtrate was collected. The filtrate was concentrated, recrystallized, filtered, and dried to yield 13.2 g of Compound C, with a yield of 68.1%. Purity: 98.1% (see FIG. 1); Chiral Purity: 99.8% (see FIG. 2).

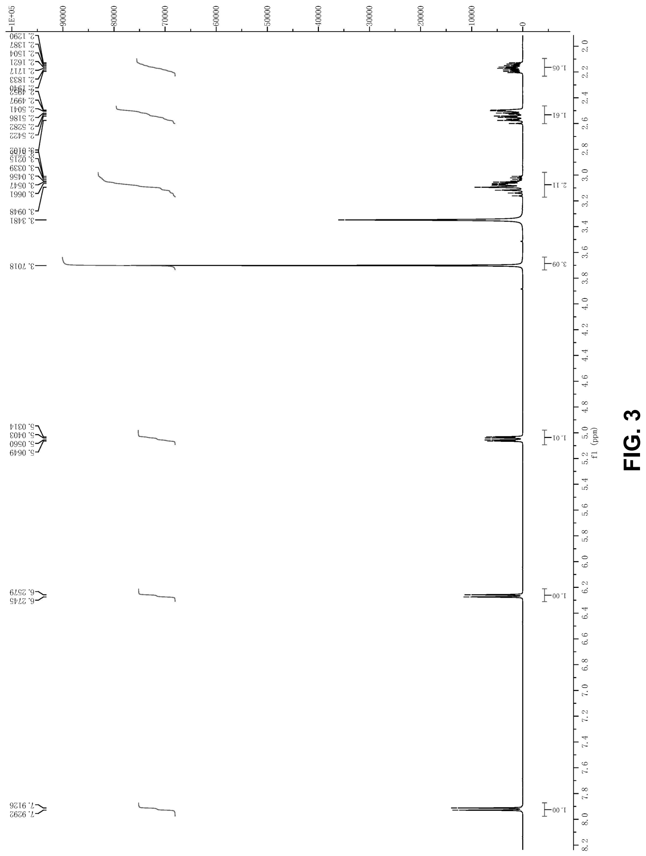

1H NMR (DMSO-d6, 400MHz): δ 7.92 (1H, d, J = 6.64 Hz); δ 6.27 (1H, d, J = 6.64 Hz); δ 5.05 (1H, dd, J = 9.84, 3.56 Hz); δ 3.70 (3H, s); δ 3.16-3.01 (2H, m); δ 2.60-2.52 (1H, m); δ 2.21-2.13 (1H, m) (see FIG. 3).

### Example 2

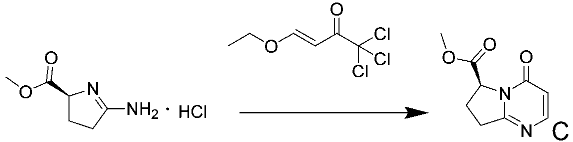

Compound E2 ((S)-5-amino-3,4-dihydro-2H-pyrrole-2-carboxylic acid methyl ester hydrochloride) (10.0 g, 56.0 mmol), toluene, Compound F-1 (1,1,1-trichloro-4-ethoxy-3-ene-2-one) (18.2 g, 83.7 mmol), and N,N-diisopropylethylamine (14.5 g, 112.2 mmol) were successively charged into a reaction vessel. The mixture was heated to 110°C to react. After the reaction was complete, the mixture was cooled to room temperature. Water was added to quench the reaction, and the phases were separated. The aqueous phase was further extracted with ethyl acetate. The combined organic phases were dried over anhydrous sodium sulfate, filtered, and the filtrate was concentrated under reduced pressure. The resultant material was recrystallized, filtered, and dried to yield 7.8 g of Compound C, with a yield of 71.7%. Purity: 98.8%; Chiral Purity: 99.6%.

### Example 3

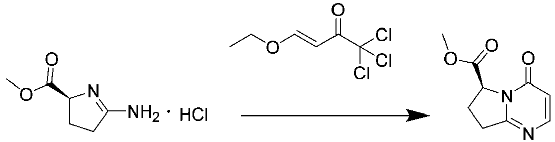

Compound E2 ((S)-5-amino-3,4-dihydro-2H-pyrrole-2-carboxylic acid methyl ester hydrochloride) (10.0 g, 56.0 mmol), ethyl acetate, Compound F-1 (1,1,1-trichloro-4-ethoxy-3-ene-2-one) (14.6 g, 67.1 mmol), and triethylamine (8.5 g, 84.0 mmol) were successively charged into a reaction vessel at ambient temperature. The mixture was heated to 50°C to react. After the reaction was completed, the mixture was cooled to ambient temperature. Water was added to quench the reaction, and the phases were separated. The aqueous phase was extracted with ethyl acetate. The combined organic phases were dried over anhydrous sodium sulfate, filtered, and the filtrate was collected. The filtrate was concentrated, recrystallized, filtered, and dried to yield 8.0 g of Compound C, with a yield of 73.6%. Purity: 99.2%; Chiral Purity: 99.7%.

### Example 4

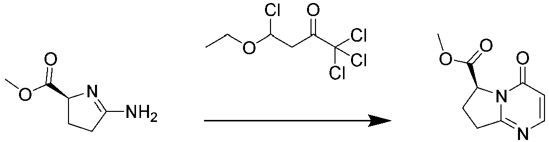

Under an inert atmosphere, trichloroacetyl chloride (34 mL, 0.3 mol) and vinyl ethyl ether (58 mL, 0.6 mol) were successively added to a reaction vessel. The mixture was stirred at room temperature for 24 hours. Unreacted trichloroacetyl chloride and vinyl ethyl ether were removed by concentration under reduced pressure, and the resulting residue was used directly as Compound F-2-2-2 (X is Cl). Toluene was added to the reaction vessel, and the mixture was stirred to dissolve Compound F-2-2-2 (X is CI), providing a toluene solution of F-2-2-2. To this solution, Compound E1 ((S)-5-amino-3,4-dihydro-2H-pyrrole-2-carboxylic acid methyl ester) (71 g, 0.50 mol) was added, followed by the dropwise addition of triethylamine (132 g, 1.2 mol). The mixture was heated to 60°C to react. After the reaction was complete, the mixture was cooled to room temperature. Water was added to quench the reaction, and the phases were separated. The aqueous phase was extracted with toluene. The combined organic phases were dried over anhydrous sodium sulfate, filtered, and the filtrate was collected. The filtrate was concentrated, recrystallized, filtered, and dried to yield 58.7 g of Compound C, with a yield of 60.5%. Purity: 97.2%; Chiral Purity: 99.0%.

### Example 5

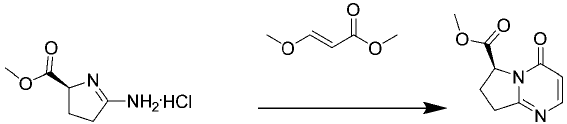

Compound E2 ((S)-5-amino-3,4-dihydro-2H-pyrrole-2-carboxylic acid methyl ester hydrochloride) (18.0 g, 101.1 mmol), toluene, Compound G-1 (methyl 3-methoxyacrylate) (15.2 g, 130.9 mmol), and triethylamine (17.3 g, 171.0 mmol) were successively charged into a reaction vessel. The mixture was heated to 90°C to react. After the reaction was complete, the mixture was cooled to room temperature. Water was added to quench the reaction, and the phases were separated. The aqueous phase was extracted with ethyl acetate. The combined organic phases were dried over anhydrous sodium sulfate, filtered, and the filtrate was concentrated under reduced pressure. The resultant material was recrystallized, filtered, and dried to yield 12.3 g of Compound C, with a yield of 62.9%. Purity: 98.7%; Chiral Purity: 99.1%.

### Example 6

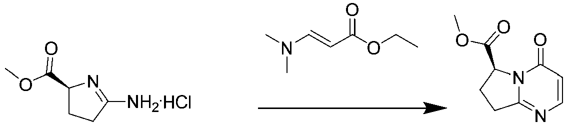

Compound E2 ((S)-5-amino-3,4-dihydro-2H-pyrrole-2-carboxylic acid methyl ester hydrochloride) (15.0 g, 84.0 mmol), ethyl acetate, Compound G-2 (N,N-dimethylaminoacrylic acid ethyl ester) (18.0 g, 125.7 mmol), and triethylamine (15.3 g, 151.2 mmol) were successively added to a reaction vessel at room temperature. The mixture was heated to 80°C to react. After completion of the reaction, the mixture was cooled to room temperature. Water was added to quench the reaction, and the phases were separated. The aqueous phase was further extracted with ethyl acetate. The combined organic phases were dried over anhydrous sodium sulfate, filtered, and the filtrate was collected. The filtrate was concentrated, recrystallized, filtered, and dried to yield 7.5 g of Compound C, with a yield of 46.0%. Purity: 97.6%; Chiral Purity: 99.3%.

### Example 7

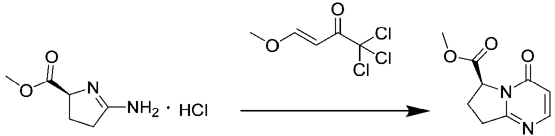

Compound E2 ((S)-5-amino-3,4-dihydro-2H-pyrrole-2-carboxylic acid methyl ester hydrochloride) (10.0 g, 56.0 mmol), toluene, Compound F-1 (1,1,1-trichloro-4-methoxy-3-ene-2-one) (17.0 g, 84.0 mmol), and N,N-diisopropylethylamine (14.5 g, 112.2 mmol) were successively charged into a reaction vessel. The mixture was heated to 110°C to react. After the reaction was complete, the mixture was cooled to room temperature. Water was added to quench the reaction, and the phases were separated. The aqueous phase was extracted with ethyl acetate. The combined organic phases were dried over anhydrous sodium sulfate, filtered, and the filtrate was concentrated under reduced pressure. The resultant material was recrystallized, filtered, and dried to yield 7.5 g of Compound C, with a yield of 70.8%. Purity: 97.9%; Chiral Purity: 99.7%.

### Example 8

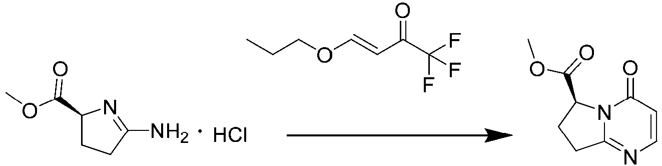

Compound E2 ((S)-5-amino-3,4-dihydro-2H-pyrrole-2-carboxylic acid methyl ester hydrochloride) (10.0 g, 56.0 mmol), toluene, Compound F-1 (1,1,1-trifluoro-4-propoxy-3-ene-2-one) (14.3 g, 78.6 mmol), and triethylamine (10.8 g, 106.7 mmol) were successively charged into a reaction vessel. The mixture was heated to 90°C to react. After the reaction was complete, the mixture was cooled to room temperature. Water was added to quench the reaction, and the phases were separated. The aqueous phase was extracted with ethyl acetate. The combined organic phases were dried over anhydrous sodium sulfate, filtered, and the filtrate was concentrated under reduced pressure. The resultant material was recrystallized, filtered, and dried to yield 7.5 g of Compound C, with a yield of 70.6%. Purity: 97.5%; Chiral Purity: 99.5%.

### Example 9

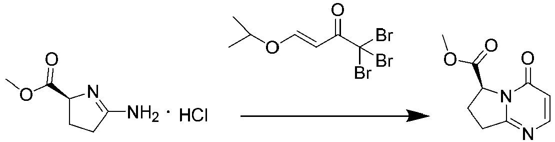

Compound E2 ((S)-5-amino-3,4-dihydro-2H-pyrrole-2-carboxylic acid methyl ester hydrochloride) (10.0 g, 56.0 mmol), toluene, Compound F-1 (1,1,1-tribromo-4-isopropoxybut-3-en-2-one) (26.4 g, 73.0 mmol), and triethylamine (10.2 g, 101.1 mmol) were successively charged into a reaction vessel. The mixture was heated to 80°C to react. After the reaction was complete, the mixture was cooled to room temperature. Water was added to quench the reaction, and the phases were separated. The aqueous phase was extracted with ethyl acetate. The combined organic phases were dried over anhydrous sodium sulfate, filtered, and the filtrate was concentrated under reduced pressure. The resultant material was recrystallized, filtered, and dried to yield 7.2 g of Compound C, with a yield of 68.9%. Purity: 95.7%; Chiral Purity: 99.4%.

### Example 10

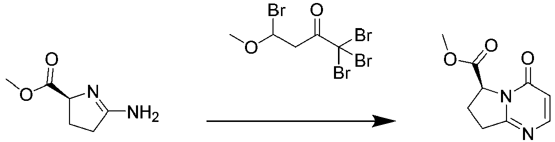

Under an inert atmosphere, toluene was added to a reaction vessel, followed by the addition of 1,1,1,4-tetrabromo-4-methoxybutan-2-one (310.1 g, 0.75 mol). Compound E1 ((S)-5-amino-3,4-dihydro-2H-pyrrole-2-carboxylic acid methyl ester) (71 g, 0.50 mol) was then added, followed by the dropwise addition of triethylamine (151.7 g, 1.5 mol). The mixture was heated to 70°C to react. After the reaction was complete, the mixture was cooled to room temperature. Water was added to quench the reaction, and the phases were separated. The aqueous phase was extracted with toluene. The combined organic phases were dried over anhydrous sodium sulfate, filtered, and the filtrate was collected. The filtrate was concentrated, recrystallized, filtered, and dried to yield 54.4 g of Compound C, with a yield of 58.3%. Purity: 96.2%; Chiral Purity: 97.5%.

### Example 11

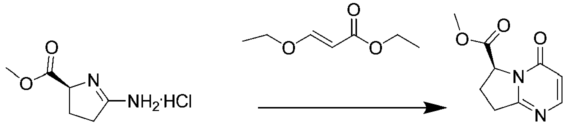

Compound E2 ((S)-5-amino-3,4-dihydro-2H-pyrrole-2-carboxylic acid methyl ester hydrochloride) (10.0 g, 56.1 mmol), toluene, Compound G-1 (3-methoxyacrylic acid ethyl ester) (12.1 g, 84.2 mmol), and triethylamine (11.4 g, 112.3 mmol) were successively charged into a reaction vessel. The mixture was heated to 50°C to react. After the reaction was complete, the mixture was cooled to room temperature. Water was added to quench the reaction, and the phases were separated. The aqueous phase was extracted with ethyl acetate. The combined organic phases were dried over anhydrous sodium sulfate, filtered, and the filtrate was concentrated under reduced pressure. The resultant material was recrystallized, filtered, and dried to yield 6.98 g of Compound C, with a yield of 65.3%. Purity: 98.1%; Chiral Purity: 99.2%.

### Example 12

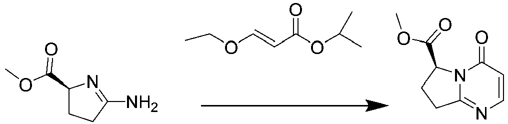

Compound E1 ((S)-5-amino-3,4-dihydro-2H-pyrrole-2-carboxylic acid methyl ester) (71 g, 0.5 mol), toluene, Compound G-1 (isopropyl 3-ethoxyacrylate) (119 g, 0.75 mol), and triethylamine (76 g, 0.75 mol) were successively charged into a reaction vessel. The mixture was heated to 90°C to react. After the reaction was complete, the mixture was cooled to room temperature. Water was added to quench the reaction, and the phases were separated. The aqueous phase was extracted with ethyl acetate. The combined organic phases were dried over anhydrous sodium sulfate, filtered, and the filtrate was concentrated under reduced pressure. The resultant material was recrystallized, filtered, and dried to yield 49.7 g of Compound C, with a yield of 52.3%. Purity: 97.9%; Chiral Purity: 98.9%.

### Example 13

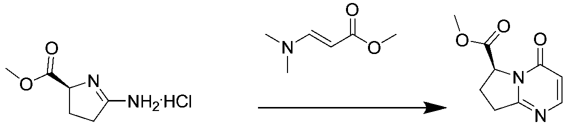

Compound E2 ((S)-5-amino-3,4-dihydro-2H-pyrrole-2-carboxylic acid methyl ester hydrochloride) (10.0 g, 56.1 mmol), ethyl acetate, Compound G-2 (methyl 3-(dimethylamino)acrylate) (10.9 g, 84.2 mmol), and triethylamine (8.52 g, 84.2 mmol) were successively added to a reaction vessel at room temperature. The mixture was heated to 80°C to react. After completion of the reaction, the mixture was cooled to room temperature. Water was added to quench the reaction, and the phases were separated. The aqueous phase was further extracted with ethyl acetate. The combined organic phases were dried over anhydrous sodium sulfate, filtered, and the filtrate was collected. The filtrate was concentrated, recrystallized, filtered, and dried to yield 6.8 g of Compound C, with a yield of 63.4%. Purity: 98.2%; Chiral Purity: 99.3%.

### Example 14

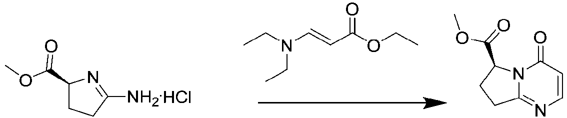

Compound E2 ((S)-5-amino-3,4-dihydro-2H-pyrrole-2-carboxylic acid methyl ester hydrochloride) (10.0 g, 56.1 mmol), ethyl acetate, Compound G-2 (ethyl 3-(diethylamino)acrylate) (9.61 g, 56.1 mmol), and triethylamine (11.4 g, 112.3 mmol) were successively added to a reaction vessel at room temperature. The mixture was heated to 90°C to react. After completion of the reaction, the mixture was cooled to room temperature. Water was added to quench the reaction, and the phases were separated. The aqueous phase was further extracted with ethyl acetate. The combined organic phases were dried over anhydrous sodium sulfate, filtered, and the filtrate was collected. The filtrate was concentrated, recrystallized, filtered, and dried to yield 5.62 g of Compound C, with a yield of 52.4%. Purity: 98.5%; Chiral Purity: 99.0%.

### Comparison Example (WO2013062881E1)

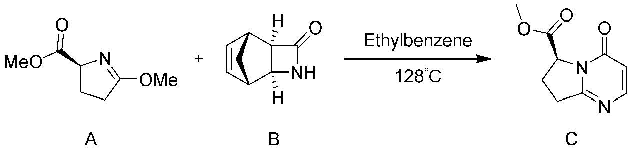

Compound A (6.60 kg, 72.3 wt. %), Compound B (4.19 kg), and ethylbenzene (9.54 L) were added to a 50 L reaction vessel. The mixture was reacted at 128°C for 48 hours with stirring. The reaction mixture was then cooled to 35°C, and toluene (14.3 L) and activated carbon (1.43 kg) were added. The mixture was maintained at 35°C and stirred for 1 hour. The mixture was filtered, and the filter cake was washed with toluene (19.1 L). The combined filtrates were concentrated. The resultant material was purified by column chromatography using silica gel (22.5 kg) and an acetone/heptane mixed solvent as the eluent. The product-containing fractions were collected and concentrated. The residue was crystallized from a solvent system of ethyl acetate/methyl tert-butyl ether (1: 4 v/v). The mixture was stirred at 5-10°C for 1 hour and then centrifuged. The solid obtained was dried under vacuum to afford Product C (2.57 kg). Yield: 44%. Purity: >99%.

It will be obvious to those skilled in the art that changes and modifications may be made, and therefore, the aim in the appended claims is to cover all such changes and modifications.

## Claims

1. A method for preparing an intermediate of an adrenergic receptor agonist, comprising contacting a first compound of formula E1 or E2 with a second compound of formula F0 to yield a third compound of formula C: 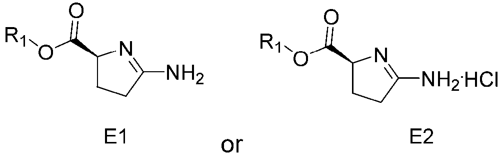 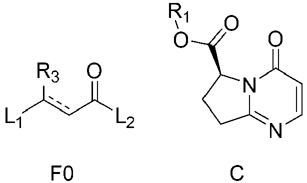 wherein:
R₁ is selected from the group consisting of methyl, ethyl, isopropyl, propyl, tert-butyl, and butyl;
R₃ is selected from the group consisting of H, Cl, F, and Br; and
L₁ and L₂ are each independently a leaving group.

2. The method of claim 1, wherein the formula F0 comprises a compound of formula F: 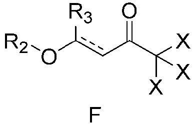
R₂ is selected from the group consisting of methyl, ethyl, isopropyl, propyl, tert-butyl, and butyl;
R₃ is selected from the group consisting of H, Cl, F, and Br; and
X is selected from the group consisting of F, Cl, and Br.

3. The method of claim 2, wherein the second compound of formula F0 comprises a compound of formula F-1: 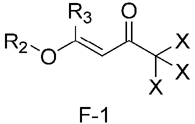
R₂ is selected from the group consisting of methyl, ethyl, isopropyl, propyl, tert-butyl, and butyl;
R₃ is H; and
X is selected from the group consisting of F, Cl, and Br.

4. The method of claim 3, wherein the compound of formula F-1 comprises the following compounds: 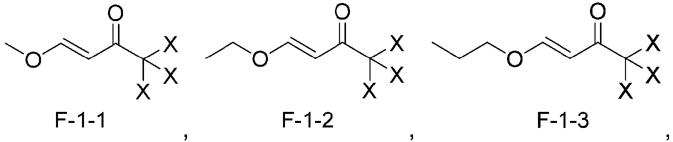 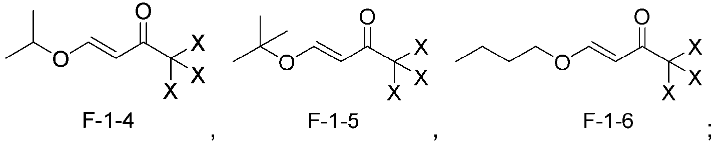 X is selected from the group consisting of F, Cl, and Br.

5. The method of claim 2, wherein the second compound of formula F0 comprises a compound of formula F-2: 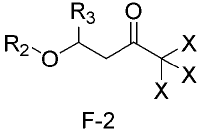
R₂ is selected from the group consisting of methyl, ethyl, isopropyl, propyl, tert-butyl, and butyl;
R₃ is selected from the group consisting of F, Cl, and Br; and
X is selected from the group consisting of F, Cl, and Br.

6. The method of claim 5, wherein the compound of formula F-2 comprises the following compounds: 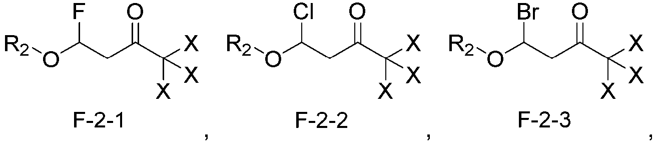
R₂ is selected from the group consisting of methyl, ethyl, isopropyl, propyl, tert-butyl, and butyl; and
X is selected from the group consisting of F, Cl, and Br.

7. The method of claim 6, wherein the compound of formula F-2-1 comprises the following compounds: 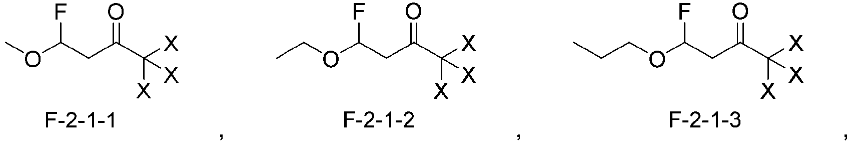 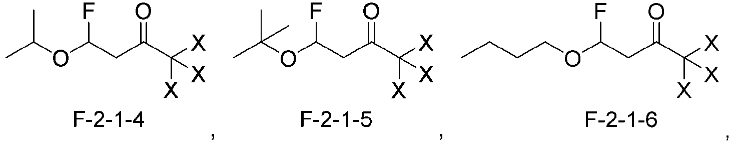 X is selected from the group consisting of F, Cl, and Br.

8. The method of claim 6, wherein the compound of formula F-2-2 comprises the following compounds: 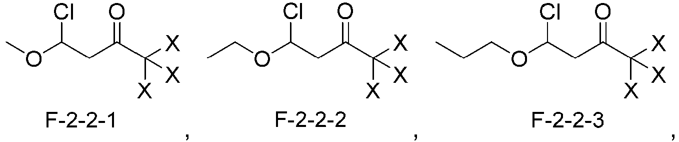 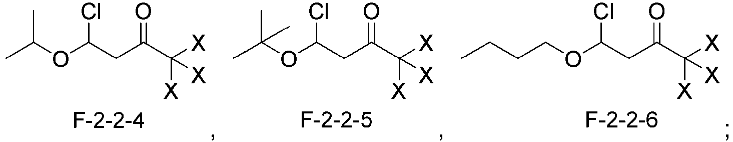 X is selected from the group consisting of F, Cl, and Br.

9. The method of claim 6, wherein the compound of formula F-2-3 comprises the following compounds: 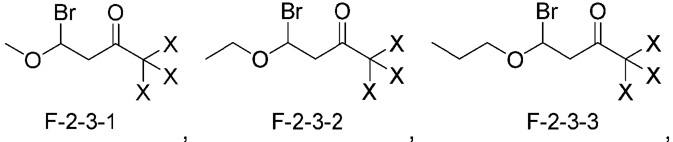 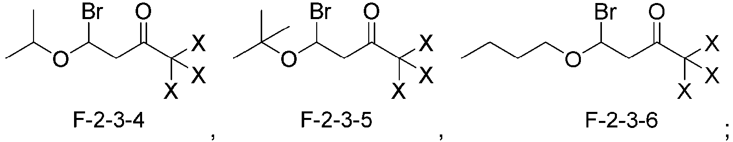 X is selected from the group consisting of F, Cl, and Br.

10. The method of claim 1, wherein the formula F comprises a compound of formula F₁: 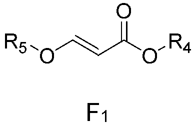 R₄ and R₅ are each independently C₁-C₆ alkyl.

11. The method of claim 10, wherein R₄ and R₅ of the compound of formula F₁ are each independently selected from the group consisting of methyl, ethyl, propyl, butyl, isopropyl, and tert-butyl.

12. The method of claim 1, wherein the formula F comprises a compound of formula F₂: 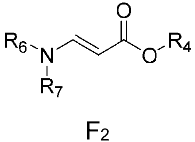 R₄, R₆, and R₇ are each independently C₁-C₆ alkyl.

13. The method of claim 12, wherein R₄, R₆, and R₇ of the compound of formula F₂ are each independently selected from the group consisting of methyl, ethyl, propyl, butyl, isopropyl, and tert-butyl.

14. The method of any one of claims 1-13, wherein the first compound of formula E1 or E2 reacts with the second compound of formula F0 in the presence of a base.

15. The method of claim 14, wherein the base is an organic base or an inorganic base.

16. The method of claim 15, wherein the organic base is selected from the group consisting of triethylamine, pyridine, N,N-diisopropylethylamine, and 4-dimethylaminopyridine.

17. The method of claim 16, wherein the organic base is triethylamine or N,N-diisopropylethylamine.

18. The method of claim 17, wherein the first compound of formula E1 or E2, the second compound of formula F0, and the base are mixed in a molar ratio in the range of 1.0: from 0.5 to 3.0: from 0.5 to 5.0.

19. The method of claim 18, wherein the first compound of formula E1 or E2, the second compound of formula F0, and the base are mixed in a molar ratio in the range of 1.0: from 1.0 to 1.5: from 1.0 to 4.0.

20. The method of claim 19, wherein the first compound of formula E1, the compound of formula F-1, and the base are mixed in a molar ratio in the range of 1.0: from 0.5 to 2.0: from 0.5 to 2.0; and the first compound of formula E2, the compound of formula F-1, and the base are mixed in a molar ratio in the range of 1.0: from 0.5 to 2.0: from 0.5 to 3.0.

21. The method of claim 20, wherein the first compound of formula E1, the compound of formula F-1, and the base are mixed in a molar ratio in the range of 1.0: from 1.0 to 1.5: from 1.0 to 1.5; and the first compound of formula E2, the compound of formula F-1, and the base are mixed in a molar ratio in the range of 1.0: from 1.0 to 1.5: from 1.5 to 2.0.

22. The method of claim 21, wherein the first compound of formula E1, the compound of formula F-2, and the base are mixed in a molar ratio in the range of 1.0: from 0.5 to 2.0: from 1.5 to 4.0; and the first compound of formula E2, the compound of formula F-2, and the base are mixed in a molar ratio in the range of 1.0: from 0.5 to 2.0: from 0.5 to 5.0.

23. The method of claim 22, wherein the first compound of formula E1, the compound of formula F-2, and the base are mixed in a molar ratio in the range of 1.0: from 1.0 to 1.5: from 2.0 to 3.0; and the first compound of formula E2, the compound of formula F-2, and the base are mixed in a molar ratio in the range of 1.0: from 1.0 to 1.5: from 2.5 to 4.0.

24. The method of claim 23, wherein
the first compound of formula E1, the compound of formula F₁, and the base are mixed in a molar ratio in the range of 1.0: from 0.5 to 2.0: from 0.5 to 2.0;
the first compound of formula E2, the compound of formula F₁, and the base are mixed in a molar ratio in the range of 1.0: from 0.5 to 2.0: from 0.5 to 3.0;
the first compound of formula E1, the compound of formula F₂, and the base are mixed in a molar ratio in the range of 1.0: from 0.5 to 2.0: from 0.5 to 2.0; and
the first compound of formula E2, the compound of formula F₂, and the base are mixed in a molar ratio in the range of 1.0: from 0.5 to 2.0: from 0.5 to 3.0.

25. The method of claim 24, wherein
the first compound of formula E1, the compound of formula F₁, and the base are mixed in a molar ratio in the range of 1.0: from 1.0 to 1.5: from 1.0 to 1.5;
the first compound of formula E2, the compound of formula F₁, and the base are mixed in a molar ratio in the range of 1.0: from 1.0 to 1.5: from 1.5 to 2.0;
the first compound of formula E1, the compound of formula F₂, and the base are mixed in a molar ratio in the range of 1.0: from 1.0 to 1.5: from 1.0 to 1.5; and
the first compound of formula E2, the compound of formula F₂, and the base are mixed in a molar ratio in the range of 1.0: from 1.0 to 1.5: from 1.5 to 2.0.

26. The method of claim 25, wherein a reaction temperature is between 25 and 120°C.

27. The method of claim 26, wherein the reaction temperature is between 50 and 110°C.

28. The method of claim 27, wherein an organic solvent is selected from the group consisting of ethyl acetate, isopropyl acetate, toluene, and acetonitrile.
